# EUROPEAN PATENT APPLICATION

(11) **EP 4 846 956 A1**
(43) Date of publication of application: **02.12.2026**
(21) Application number: 25178847.7
(22) Date of filing: 26.05.2025
(51) Int. Cl.: A61B 17/32, A61B 17/00, A61B 90/00

(54) **MORCELLATOR**

(71) Applicant: WISAP Medical Technology GmbH, 85649 Brunnthal (DE)
(72) Inventor: HILGERS, Stephan, 85649 Brunnthal (DE); VOLK, Jonas, 85649 Brunnthal (DE)
(74) Representative: Grünecker Patent- und Rechtsanwälte PartG mbB

(57) **Abstract**

The invention relates to a morcellator (1, 100) comprising: a rotatable cutting tube (2) with a cutting edge (3) for cutting and removing human tissue; a handle (4) for handling the morcellator (1, 100), wherein the cutting tube (2) is rotatably connected with the handle (4); and a motor (12) for rotatably driving the cutting tube (2). The motor (12) is reversibly connectable with the handle (4) in a predefined position, wherein the motor (12) comprises a motor positioning surface (19, 119) and wherein the handle (4) comprises a handle positioning surface (12, 120), wherein the motor positioning surface (19, 119) is configured for being in contact with the handle positioning surface (12, 120) in the predefined position for aligning the motor (12) with respect to the handle (4).

## Description

The present invention relates to a morcellator.

In minimally invasive surgical procedures such as laparoscopic and robot-assisted surgery, large masses of tissue often have to be removed. For example, a surgeon may need to remove a uterine fibroid from an abdominal cavity without making a large incision in the patient. Laparoscopy is a surgical procedure using small incisions each less than about 15 millimeters in diameter to gain access to an internal surgical site. Morcellators assist surgeons in severing and removing large tissue masses from the patient in minimally invasive surgery.

Traditionally, a morcellator consists of a rotating cylindrical inner tube with a sharp distal cutting edge that rotates in a stationary outer tube. The morcellator is inserted into the patient via a cannula or trocar or directly via an incision. The surgeon inserts the grasping instrument (i.e., tenaculum) through the cylindrical tube, grasps the tissue to be morcellated and pulls it through the inner lumen of the tube. As the grasper or tenaculum pulls the tissue through the tube, the tissue is positioned at the cutting edge or circumference of the tube, which cuts, severs or mor-cellates the tissue. Laparoscopic surgeries are often performed in stages. By repeating the grasping and severing process, the surgeon can incrementally remove the large mass of tissue.

Usually, the cylindrical inner tube with the sharp distal cutting edge is rotated by a motor that is arranged in a hand module of the morcellator. The hand module, the cylindrical inner tube and the motor are usually permanently connected so that the morcellator can be used directly for operation without the need of prior assembling the morcellator.

However, a disadvantage is that the cylindrical inner tube is a disposable. Thus, the entire morcellator can only be used for a single medical treatment. Thus, the resources used for the morcellator are not utilised efficiently and the morcellator as a whole is unsustainable. Further, replacing the entire morcellator every time a new surgical step is performed, is expensive.

Further, there is a risk that the cut tissue, which is transported through the rotating cylindrical inner tube is damaged during transport. In the worst case, the tissue is torn, deposits on the inner walls of the inner tube and clogs the inner tube. The operation must then be stopped, which poses an increased health risk for the patient. Potentially, a new morcellator must be used. This makes morcellators inefficient with respect to cost and time.

It is desirable to provide a sustainable and cost efficient morcellator, which allows precisely removing a defined amount of tissue in a time and cost efficient manner.

A first aspect of the invention relates to a morcellator comprising: a rotatable cutting tube with a cutting edge for cutting and removing human tissue; a handle for handling the morcellator, wherein the cutting tube is rotatably connected with the handle; and a motor for rotatably driving the cutting tube, wherein the motor is reversibly connectable with the handle in a predefined position, wherein the motor comprises a motor positioning surface and wherein the handle comprises a handle positioning surface, wherein the motor positioning surface is configured for being in contact with the handle positioning surface in the predefined position for aligning the motor with respect to the handle.

The motor may be reusable, while the handle with the cutting tube may be disposable. Thus, providing sterilized equipment may be facilitated by using a new sterilized handle with sterilized cutting tube for each surgery, while reusing the motor.

In particular, aligning the motor involves that the motor positioning surface and the handle positioning surface are only in contact when the motor is in an aligned position with respect to the handle. Aligning the motor may comprise rotatory and translational alignment. The rotary alignment may be around the translational alignment direction. The translational alignment may be in the direction of a drive shaft of the motor and the rotational alignment may be a rotation around the drive shaft of the motor.

In particular, the motor positioning surface and the handle positioning surface differ from a surface normal to the direction of a drive shaft of the motor or a insertion direction of the motor into the handle. This difference may be inclination or structure or both.

It is particularly easy for the operator of the morcellator to connect the motor to the handle in the correct manner. In particular, the motor positioning surface is adapted to fit to the handle positioning surface only in the predefined position, when the motor and handle are correctly connected. Further, this may prevent that an incompatible motor is connected to a handle. Thus, the operator does not have to try different connection orientations for connecting the handle and the motor. Consequently, there is no time delay when assembling the morcellator. Further, it may be prevented that the motor or handle are inoperable or damaged, when in an incorrect relative orientation.

The cutting tube may comprise a proximal end proximal to the handle, and a distal end with the cutting edge distal from the handle. The cutting tube may at least partially extend through the handle. At the proximal end, the cutting tube may be, in particular irreversibly, fixed to the handle. Generally, cleaning of the cutting tube after an operation may be not possible, because medical hygiene standards must be adhered to. As a consequence, a cutting tube may only be used once. If the cutting tube is irreversibly fixed to the handle, this set of handle and cutting tube may be configured for single use. In contrast, the motor may be configured for multiple use. The morcellator according to the invention may be more sustainable, since the motor may be detached from the handle and be used several times with other handles.

The cutting tube may be configured for being rotatable around an axis of rotation. The axis of rotation of the cutting tube may be a central longitudinal axis of the cutting tube, in particular extending from the proximal end to the distal end. A distal direction may be a direction away from the handle along the axis of rotation. A proximal direction may be a direction against the distal direction, i.e. towards the handle along the axis of rotation.

The proximal end and the distal end may be open ends. The cutting tube may be a hollow tube. At the proximal end, the cutting tube may have a proximal (inner) diameter. At the distal end, the cutting tube may have a distal (inner) diameter.

The handle may extend in an axial direction along a longitudinal axis of the handle. The longitudinal axis of the handle may be the main extension direction of the handle. Preferably, the longitudinal axis of the handle may be inclined in an angle of at least 45 degrees, in particular at least 60 degrees, in particular at least 80 degrees with respect to the axis of rotation of the cutting tube. The longitudinal axis of the handle may be substantially perpendicular to the axis of rotation of the cutting tube. The handle may be formed of a polymer.

The motor may extend in a longitudinal direction along a longitudinal axis. In a connected state, the longitudinal axis of the motor may be parallel to, in particular coincide with, the longitudinal axis of the handle.

Preferably, a rotary movement between the motor and the handle is blocked by the contact of the motor positioning surface and the handle positioning surface, when the motor is connected to the handle. The rotary movement may describe a relative movement of the motor and the handle around the longitudinal axis of the handle and/or the motor. Thus, the motor may be prevented from being twisted or rotationally dislocated relative to the handle in a connected state. This may reduce the risk of damaging the motor or handle during use. Further, this may ensure safe use of the morcellator by the operator.

Preferably, the motor positioning surface is in a form-fit with the handle positioning surface, when the motor is connected to the handle. This may allow a precise and secure connection of the handle and the motor. The form-fit connection may be a mechanical connection of the motor positioning surface and the handle positioning surface. The motor positioning surface and the handle positioning surface may interlock or fit together due to their geometric shapes, in particular surface structures. Furthermore, a form-fit connection may be detachable. Thus, the motor may be easily removed from the handle.

Preferably, the motor comprises a drive shaft configured for driving the cutting tube, wherein the motor comprises a motor housing extending at least partially around the drive shaft, and wherein the motor housing comprises the motor positioning surface.

The motor positioning surface may extend to an outer surface of morcellator. This may allow quick and easy assembly of the motor and the handle. Since the motor housing is a component that is already required on the morcellator, by providing the motor positioning surface on the motor housing, no further separate component having the motor positioning surface is required. Thus, the number of components and complexity of the morcellator as well as manufacturing costs may be reduced. Further, the motor housing may prevent the operator of the morcellator from contacting the rotating drive shaft.

The motor housing may be formed of stainless steel or titanium.

The drive shaft may be connected to a drive via a motor cable for rotating the drive shaft. The drive shaft may be axially fixed within the motor housing. The drive shaft may be rotatable within the motor housing. A motor bevel gear may be mounted to the drive shaft and may be rotatable with the drive shaft. The cutting tube may comprise a tube bevel gear mounted to the cutting tube. Optionally, the tube bevel gear may be mounted to a shaft connected to the cutting tube and which drives the cutting tube. If the motor is connected to the handle, the motor bevel gear may be perpendicular to the tube bevel gear. The motor bevel gear may drive the tube bevel gear for rotating the cutting tube. The motor bevel gear and the tube bevel gear may maintain or increase the speed of the motor rotation when driving the cutting tube.

Preferably, the handle extends in an axial direction, wherein the handle has a motor receiving cavity, wherein the motor is insertable into the motor receiving cavity. Thus, the motor may be covered by the handle, thereby preventing the operator of the morcellator from contacting the rotating drive shaft. This may enable an even safer use of the morcellator.

The motor receiving cavity may extend in the axial direction of the handle. The motor may be insertable into the motor receiving cavity by moving the motor along the axial direction into the motor receiving cavity. By inserting the motor into the motor receiving cavity, the motor bevel gear may be contactable with the tube bevel gear.

In particular, the axial direction of the handle may is in the longitudinal axis of the handle.

Preferably, the handle positioning surface is formed by a surface of the handle adjacent to the motor receiving cavity. Since the handle is a component that is already required for the morcellator, by providing the handle positioning surface on a surface of the handle, no further separate component comprising the handle positioning surface is required.

The handle positioning surface may surround the motor receiving cavity. The handle positioning surface may have a circular or oval or rectangular form or combinations thereof, in particular when viewed from the longitudinal axis of the handle.

Alternatively, the handle positioning surface may be formed by, at least, one internal wall in the motor receiving cavity. In this case, the motor receiving cavity may for example have a substantially circular internal wall and one flat wall. The motor housing may have a complementary shape, thereby indicating the correct positioning of the motor relative to the handle. Optionally, a protrusion or groove may be formed on the internal wall of the motor receiving cavity, which may be contactable with a corresponding protrusion or groove on the motor housing.

The handle positioning surface may be integrally formed with the handle. Optionally, a separate part may be fixed, e.g. glued, to the handle, which may form or comprise the handle positioning surface. Optionally, the separate part may be connected to the handle in a force-fitting manner, e.g. by pressing it around an outer surface of the handle.

Preferably, the handle positioning surface extends in a plane that is at least locally inclined to the longitudinal axis of the handle, wherein the motor positioning surface extends in a plane that is at least locally inclined to the longitudinal axis of the motor housing, and wherein the handle positioning surface and the motor positioning surface are inclined by the same degree in corresponding locations. This may facilitate the identification of how the handle and the motor are to be connected, thereby allowing quick and easy assembling.

The handle positioning surface and/or the motor positioning surface may be inclined by at least 10 degrees, at least 30 degrees, at least 45 degrees. The handle positioning surface and/or the motor positioning surface may be inclined by at most 90 degrees, 80 degrees, or 60 degrees.

The motor positioning surface may have an increased diameter relative to the remaining motor housing. This may increase the contact surface, thereby enabling a secure connection of the motor positioning surface and the handle positioning surface. In addition, this may help the user to identify the motor positioning surface and quickly identify how to connect the motor to the handle in the correct manner.

The diameter of the motor positioning surface may depend on of the handle positioning surface, in particular the diameter of the handle or its motor receiving cavity. A diameter of the motor positioning surface may be at least 1 millimetre, preferably 3 millimetres, and/or at most 10 millimetres, preferably at most 5 millimetres larger than the diameter of the motor housing. The motor positioning surface may have a circular or oval or rectangular form or combinations thereof, in particular when viewed from the longitudinal axis of the motor housing.

The motor positioning surface may be integrally formed with the motor housing. For example, the motor positioning surface may be formed by a circumferential outer wall of the motor housing. Optionally, a separate part may be, in particular torque-proof, fixed, e.g. glued, to the motor housing, which may form or comprise the motor positioning surface. Optionally, the separate part may be connected to the motor housing in a force-fitting manner, e.g. by pressing it around an outer surface of the motor housing.

Preferably, the motor positioning surface has a repeating structure, in particular extending in the circumferential direction of the motor housing, and wherein the handle positioning surface has a repeating structure, in particular extending in the circumferential direction of the handle. Due to the repeating structure, more than one predefined positions for aligning the motor with respect to the handle may be possible. By rotating the motor with respect to the handle (or vice versa), the motor positioning surface may be contacted with the handle positioning surface in one of these predefined positions. This may allow quick and easy assembling. The repeating structure may extend around the whole or part of the motor housing or the handle housing in the circumferential direction.

The motor positioning surface and the handle positioning surface may be structured surfaces. The motor positioning surface may be complementary to the handle positioning surface. This may facilitate the identification of how the handle and the motor are to be connected, therefore allowing quick and easy assembling.

For example, the motor positioning surface or the handle positioning surface may comprise at least one recess and/or protrusion, which may be contactable with a corresponding recess and/or protrusion of the other of the motor positioning surface and the handle positioning surface. The recess and/or protrusion may have a rectangular, polygonal, rounded shape or combinations thereof. Alternatively or optionally, the handle positioning surface and the motor positioning surface may be, at least partially, flat surfaces. Thus, the manufacturing costs of the morcellator may be reduced.

Preferably, the morcellator comprises a cap for, in particular axially, fixing the motor with respect to the handle in a detachable manner. The cap may secure the axial connection of the handle positioning surface and the motor positioning surface. An axial fixation may mean that the motor may not be removed from the handle (or vice versa) along the longitudinal axis of the handle or the longitudinal axis of the motor housing if the cap is fixed to the handle. The motor may be fixed to the handle by first connecting the motor positioning surface to the handle positioning surface in the predefined position and then fixing the cap to the handle.

The cap may be formed of plastic or stainless steel or titanium. The cap may comprise an opening through which the motor cable connecting the drive with the drive shaft for rotating the drive shaft may be guided. The opening may be smaller than a diameter of the motor housing such that the cap may firmly hold the motor housing to the handle.

The cap may be configured for being fixed to the handle by a threaded connection. For example, the cap may be a screw nut. The cap may be hollow, comprising an internal thread. The handle may comprise a corresponding external thread, which may be screwed with the internal thread. Optionally, the cap may comprise a magnet and the handle may comprises a magnet, wherein the cap may be fixable to the handle by the magnets. The cap may be fixable to the handle by means of a screw. The cap may be fixable to the handle by means of a clamp.

A second aspect of the invention relates to a morcellator comprising: a rotatable cutting tube with a cutting edge for cutting and removing human tissue; a handle for handling the morcellator, wherein the cutting tube is rotatably connected with the handle; a motor for rotatably driving the cutting tube; and a stationary outer tube fixed to the handle, wherein the outer tube is configured to extend around the cutting tube; and an insert, wherein the insert is positioned between the outer tube and the cutting tube.

The insert may serve as buffer, guidance and/or support for the cutting tube within the outer tube. This may prevent that an inner surface of the outer tube may be damaged when the cutting tube rotates inside the outer tube.

Preferably, the insert provides a bearing in between the outer tube and the cutting tube. Thus, the insert may enable and support a relative movement of the rotatable cutting tube with respect to the stationary outer tube.

The insert may comprise a hollow cylindrical bearing part. The bearing part may be torque-proof fixed to the inner surface of the outer tube. For example, the bearing part may be glued to the inner surface of the outer tube. Optionally, the bearing part may be connected to the outer tube in a force-fitting manner, in particular pressed into the outer tube.

The insert may comprise a proximal end proximal to the handle, and a distal end distal from the handle. In particular, the proximal end of the insert is in a distance to the handle. The insert may comprise a central longitudinal axis, in particular extending from the proximal end to the distal end of the insert. At the proximal end, the insert may have a proximal (inner) diameter. At the distal end, the insert may have a distal (inner) diameter. The proximal end may have be greater than the diameter at the distal end. The insert, in particular the bearing part, may have a radial thickness of at least 0.5 millimetres, preferably at least 1 millimetre, and/or at most 3 millimetres, preferably at most 2 millimetres. The insert may have a length of at least 5 millimetres, preferably at least 10 millimetres, and/or at most 30 millimetres, preferably at most 20 millimetres, in particular measured along the axial direction. The insert may be formed of plastic.

Preferably, the cutting tube has a proximal end proximal to the handle, and a distal end with the cutting edge distal from the handle, wherein the proximal end has the same diameter as the distal end. Thus, no further manufacturing step for adjusting the shape, in particular the diameter, of the cutting tube is required, allowing the cutting tube to be manufactured in a time and cost-efficient manner.

The cutting tube may have an outer diameter of at least 7 millimetres, or at least 10 millimetres. The cutting tube may have an outer diameter of at most 18 millimetres, or at most 15 millimetres, or at most 13 millimetres.

The outer tube may comprise a proximal end proximal to the handle, and a distal end distal from the handle. The outer tube may comprise a central longitudinal axis, in particular extending from the proximal end to the distal end of the outer tube. At the proximal end, the outer tube may have a proximal diameter. At the distal end, the outer tube may have a distal diameter. The proximal end may have the same diameter as the distal end. The outer tube may have an inner diameter of at least 8 millimetres, least 12 millimetres, and/or at most 19 millimetres, at most 17 millimetres, at most 15,4 millimetres, preferably at most 15 millimetres.

The proximal end and the distal end of the outer tube may be open ends. The outer tube may be a hollow tube. At least a portion of, the outer tube may be configured to extend around the cutting tube. The proximal end of the outer tube may be, in particular irreversibly, fixed to the handle, in particular such that the outer tube is not rotatable. Thus, the outer tube may prevent the operator of the morcellator from contacting the rotating cutting tube. The outer tube may protect the operator of the morcellator from being injured by the rotating cutting tube. As will be described below, the outer tube may be adjustable, in particular positionable closer or further away from the handle, along the longitudinal axis of the outer tube.

Preferably, the insert comprises a lip, which covers, at least part of, the cutting edge and extends around the cutting edge into an inside of the cutting tube. The lip may reduce a cross-sectional area inside the cutting tube, in particular measured in a plane perpendicular to the axis of rotation of the cutting tube.

The insert may have a distal end in the form of a protrusion protruding over the distal end of the outer tube. The distal end of the insert may be provided in the region of the lip. The distal end of the insert may be flat and slanted and/or rounded.

The lip may exceed the distal end of the insert. The lip may exceed the distal end of the cutting tube. The lip may exceed the distal end of the outer tube.

If the cutting tube is rotated and the cutting edge, which is not covered by the lip contacts the tissue, the tissue may be cut. Then, the cut tissue may be transported into the cutting tube and removed out of the patient's body. With the part of the cutting edge covered by the lip, not cutting may be performed. Thus, the lip may limit the cutting edge being configured for cutting the tissue to the uncovered part of the cutting edge.

The lip may be adapted to stabilize the cut tissue against the rotating cutting tube. This may be enabled, since the lip is not rotating with the cutting tube but remains stationary with the outer tube. It may be ensured that the tissue, which is transported into the cutting tube, is not damaged, e.g. torn, during transport.

When the cut tissue contacts the insert, in particular the lip, the tissue may be automatically distanced from the inner surface of the cutting tube. Thus, that the area where the tissue contacts the rotating inner surface of the cutting tube may be reduced. The rotating cutting tube inflects less or no rotary movement on the tissue. This may reduce the risk that the tissue is torn during transport into the cutting tube. Further, segments of tissue are prevented from depositing on the inner walls of the cutting tube and clogging the cutting tube, which requires the operation to be stopped. Thus, this embodiments not only reduces the need of exchanging a blocked morcellator by a new morcellator but also reduces the health risk for the patient by preventing the operation from lasting longer because the morcellator has to be changed.

The lip may be integrally formed with the insert. The lip may be formed of a polymer or plastic. The insert may be manufactured by injection moulding. This may have the advantage that no further processing of the insert is required. The lip may be attached, in particular glued or sewed, to the bearing part.

Preferably, the lip extends along an inner surface of the cutting tube having a substantially constant thickness. Thus, the cross-sectional area inside the cutting tube may be reduced by the lip in a predefined amount.

The lip may have a radial thickness of at least 0.5 millimetres, preferably at least 1 millimetre, and/or at most 3 millimetres, preferably at most 2 millimetres. The thickness of the lip may be measured from a radial surface of the insert that faces the inside of the cutting tube, i. e. that is not in contact with the outer tube.

The lip may protrude at least 2 millimetres over a distal end of the outer tube. In particular, the lip protrudes 2 millimetres to 20 millimetres over the outer tube. In particular, the lip protrudes 3 millimetres to 10 millimetres over the outer tube. In particular, the lip protrudes 8 millimetres to 10 millimetres over the outer tube. In particular, the lip protrudes 3 millimetres to 5 millimetres over the outer tube. The lip may facilitate insertion of the morcellator into a surgical incision, in particular into an abdominal cavity.

The lip may protrude at least 1 millimetre over a distal end of the cutting tube, at least 2 millimetres over a distal end of the cutting tube, or at least 3 millimetres over a distal end of the cutting tube, in particular in the cutting position. In particular, the lip protrudes 1 millimetre to 4 millimetres over the cutting tube, or 2 millimetres to 3 millimetres over the cutting tube, in particular in the cutting position.

The lip may have a slanted or rounded form toward its end distal from the handle. This may further facilitate insertion into an incision.

The outer tube and cutting tube may be axially movable relative to each other. Preferably, the outer tube is axially movable relative to the cutting tube between a safe position and a cut position. The insert fully may fully cover the cutting tube in the safe position. This may allow the cutting tube to be safely inserted into the patient without damaging tissue. Thus, there is no need for an obturator because the insert fully covers the sharp cutting edge of the cutting tube. The insert may partially cover the cutting tube in the cut position.

The insert may be movable together with the outer tube.

If the outer tube is in the safe position the distal end of the outer tube may be further away from the handle than in the cut position. The outer tube may be axially movable along the longitudinal axis of the outer tube by at least 1 millimeter, preferably at least 3 millimeters, and/or at most 10 millimeters, preferably at most 5 millimeters. In the safe position, there may be a gap between the outer tube and the handle, corresponding to the axial movement of the outer tube. In the safe position, cutting of the tissue may not be possible.

When the outer tube is in the cut position, cutting of the tissue may be enabled.

On the handle, a rotatable cam surface may be provided and on the outer tube a complementary cam surface may be provided, which may be rotatable relative to each other for, in particular manually, for moving the outer tube relative to the cutting tube and switching between the safe position and the cut position.

During inserting the cutting tube and the outer tube into the patient, the outer tube may be in the safe position fully covering the cutting tube, in particular the cutting edge. After insertion, the outer tube may be switched, in particular retracted, to be in the cut position. Thereby the cutting edge, except for the part covered by the lip, is uncovered and may cut the tissue by rotation.

The lip may cover at least 10 percent, preferably at least 20 percent, in particular at least 30 percent, and/or at most 50 percent, preferably at most 40 percent of the cutting edge, in particular measured in a circumferential direction of the cutting edge, when the outer tube is in the safe position.

The lip may span an angle of at least 30 degrees, preferably at least 40 degree, preferably at least 520 degree, and/or at most 180 degree, preferably at most 170 degree, preferably at most 160 degree, in particular measured in a plane at the distal end of the outer tube.

The distal end of the insert may have a rounded edge. This may reduce the risk of cutting tissue during insertion of the cutting tube and the outer tube into the patient.

Features or explanations described with regard to one of the aspects (morcellators) may be transferred to and combined with the other aspects.

The invention is explained in more detail below with reference to exemplary embodiments.
Figure 1 shows a perspective view of a morcellator according to a first embodiment;
Figure 2 shows an enlarged side view of the motor and handle positioning surfaces in the morcellator according to the first embodiment;
Figure 3 shows a perspective view of a morcellator according to a second embodiment;
Figure 4 shows a side view of a morcellator in a safe position;
Figure 5 shows a side view of a morcellator in a cut position;
Figure 6 shows a perspective view of a cutting tube of a morcellator in a safe position;
Figure 7 shows a perspective view of a cutting tube of a morcellator in a cut position;
Figure 8 shows a sectional view of the outer tube, cutting tube and the insert in the safe position; and
Figure 9 shows a sectional view of the outer tube, cutting tube and the insert in the cut position.

Identical technical components are provided with the same reference signs throughout the figures.

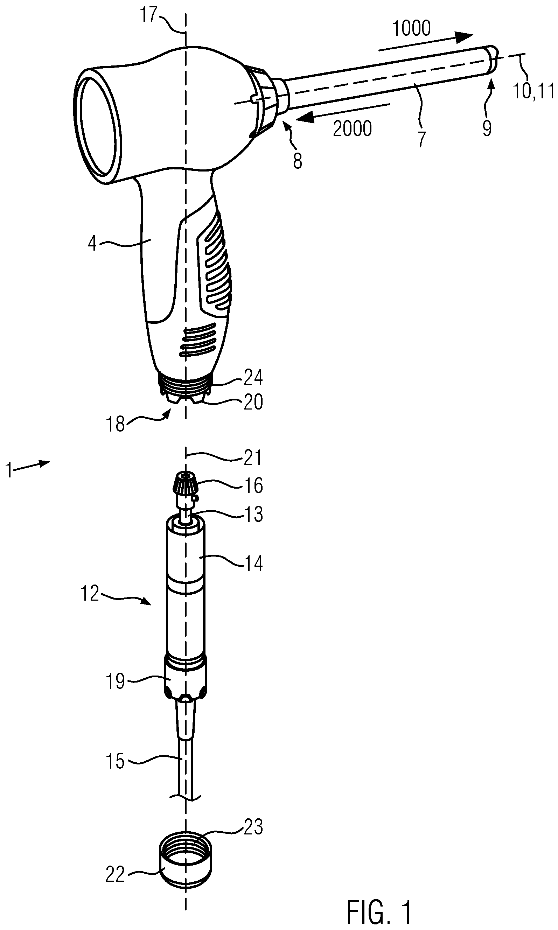

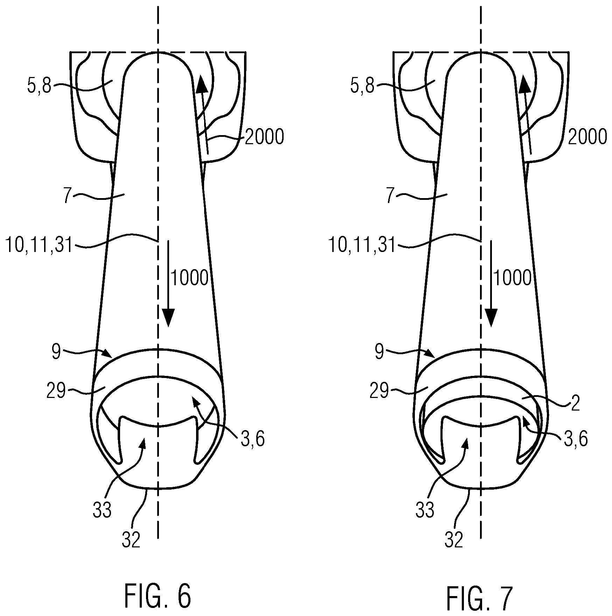

Figure 1 shows a morcellator 1 comprising a rotatable hollow cutting tube 2 with a cutting edge 3 (cf. Figures 6 to 9) for cutting and removing human tissue (not shown). Further, the morcellator 1 comprises a handle 4 for handling the morcellator 1. The cutting tube 2 extends through the handle 4 and is irreversibly fixed to the handle 4 on its proximal end 5. On a distal end 6 of the cutting tube 2, the cutting edge 3 is provided distal from the handle 4. The cutting tube 2 extends from the proximal end 5 to the distal end 6 along a longitudinal axis 10, i.e. in a distal direction 1000 away from the handle 4. A proximal direction 2000 is a direction against the distal direction 1000, i.e. towards the handle 4. A proximal (inner) diameter at the proximal end 5 is the same as a distal (inner) diameter 38 at the distal end 6, here eight millimetres. If driven by a motor 12, the cutting tube 2 rotates around the longitudinal axis 10, which is the axis of rotation.

Further, the morcellator comprises an outer hollow tube 7, which is fixed to the handle 4 on a proximal end 8 of the outer tube 7. Further, the outer tube 4 has a distal end 9 distal from the handle 4. The outer tube 7 extends around the cutting tube 2. Further, the outer tube 7 is not rotating with the cutting tube 2, thereby preventing the operator of the morcellator from contacting the rotating cutting tube 2 and thus protecting the operator from being injured by the rotating cutting tube 2. The outer tube 7 extends from the proximal end 8 to the distal end 9 along a longitudinal axis 11. A proximal diameter at the proximal end 8 is the same as a distal diameter 39 at the distal end 9, here 12 millimetres.

The longitudinal axis 10 and 11 of the cutting tube 2 and the outer tube 7 coincide, i.e. the tubes 2, 7 are arranged concentrically.

The motor 12 comprises a rotatable drive shaft 13, which is fixed to a drive (unit shown) via a motor cable 15 for rotating the drive shaft 13 with a predefined speed. A motor housing 14 extends around the drive shaft 13 in an axial direction along a longitudinal axis 21. Thus, the motor housing 14 prevents the operator of the morcellator 1 from contacting the rotating drive shaft 13.

As shown in Figure 1, the handle 4 extends in an axial direction along a longitudinal axis 17. Further, the handle 4 has a motor receiving cavity 18 into which the rotatable drive shaft 13 together with the motor housing 14 can be inserted. For rotating the cutting tube 2, a motor bevel gear 16 is mounted to the drive shaft 13. The motor bevel gear 16 is rotatable with the drive shaft 13. The cutting tube 2 comprises a tube bevel gear (not shown) on the part of the cutting tube 2 extending through the handle 4. If the motor 12, i.e. the drive shaft 13 and the motor housing 14, is inserted into the motor receiving cavity 18 of the handle 4, the motor bevel gear 16 is connected with the tube bevel gear such that the motor bevel gear drives the tube bevel gear for rotating the cutting tube 2.

Advantageously, the motor 12 is reversibly connectable with the handle 4. For each surgery, a new sterilized handle 4 with cutting tube 2 may be used. Further, the motor 12 may be replaced if it has a defect. The handle 4 with the cutting tube 2 and the outer tube 7 may be detached from the motor 12 and exchanged by another handle 4 with the cutting tube 2 and the outer tube 7. If the motor 12 is fixed to the handle 4, the longitudinal axis 17 of the handle 4 coincides with the longitudinal axis 21 of the drive shaft 13 or of the motor housing 14.

Further, the motor 12 is connectable with the handle 4 in a predefined position, which shows the operator that the motor 12 is correctly aligned respect to the handle 4. This prevents from falsely assembling the morcellator. According to the embodiment shown in Figure 1, the motor housing 14 comprises a motor positioning surface 19. The handle 4 comprises a complementary handle positioning surface 20, which is contactable with the motor positioning surface 19 if the motor 12 is in the predefined position with respect to the handle 4. I.e., if the motor 12 is not in the predefined position with respect to the handle 4, the handle positioning surface 20 is not fully or correctly contactable with the motor positioning surface 19. If the motor 12 is connected to the handle 4, a rotary movement between the motor 12 and the handle 4 is blocked by the contact of the motor positioning surface 19 and the handle positioning surface 20. In other words, the motor 12 is prevented from being twisted relative to the handle 4 around the longitudinal axis 17 of the handle 4.

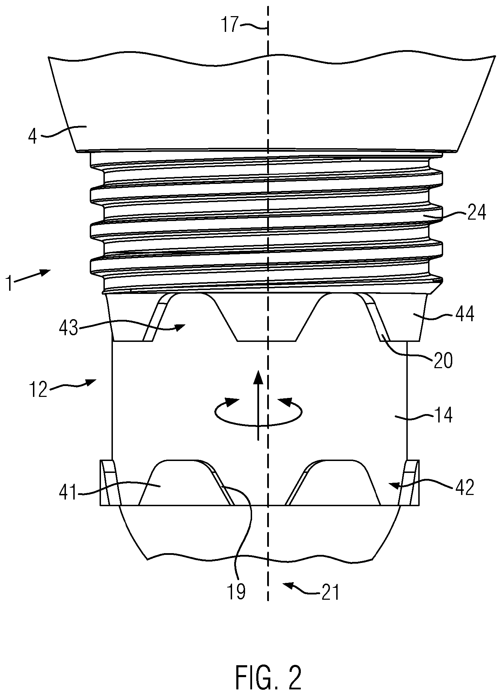

As can be seen in Figures 1 and 2, the motor positioning surface 19 is provided on an outer circumferential surface the motor housing 14. In particular, the motor positioning surface 19 is integrally formed by the motor housing 14. The motor positioning surface 19 has an increased diameter relative to the remaining motor housing 14. The motor positioning surface 19 has a repeating structure extending in the circumferential direction around the circumferential surface the motor housing 14. In the present case, the motor positioning surface 19 comprises several rounded recesses 41 and protrusions 42, which are alternately arranged on the circumferential surface the motor housing 14. From a view along the longitudinal axis 21 of the motor housing 14, the motor positioning surface 19 has a circular form.

The handle positioning surface 20 is integrally formed by a surface surrounding the motor receiving cavity 18. The handle positioning surface 20 has a repeating structure extending in a circumferential direction of the handle 4. In the present case, the handle positioning surface 20 comprises several rounded recesses 43 and protrusions 44, which are alternately arranged on the said surface of the handle 4. From a view along the longitudinal axis 17 of the handle 4, the handle positioning surface 20 has a circular form.

For aligning the motor 12 with the handle 4, the drive shaft 13 is axially moved towards the handle 4 into its motor receiving cavity 18 (or vice versa) (cf. arrow pointing upwards in Figure 2). Simultaneously, the motor 4 is rotated (clockwise or counter clockwise) around the longitudinal axis 17 of the handle 4 (or vice versa) until the motor positioning surface 19 fully contacts the handle positioning surface 20 (cf. curved arrow in Figure 2). This indicates that the motor 12 and the handle 4 are aligned in the predefined position. Thus, when the motor 12 is connected to the handle 4, the motor positioning surface 19 is in a form-fit with the handle positioning surface 20.

Due to the said repeating structure, more than one predefined positions for aligning the motor 12 with respect to the handle 4 are possible. Thus, for assembling the morcellator 1, the motor 12 only needs to be rotated until one protrusion of the motor housing 14 engages in a recess pf the handle 4.

Further, the morcellator 1 comprises a cap 22 for axially fixing the motor 12 with respect to the handle 4 in a detachable manner. For this purpose, the cap 22 is provided as a screw nut for being fixed to the handle 4 by a threaded connection. The cap 22 is hollow and comprises an internal thread 23 on its inner circumferential surface. The motor cable 15 fixed to the drive shaft 13 is guided through an opening of the cap 22. The handle 4 comprises a corresponding external thread 24. By fastening the internal thread of the cap 22 to the external thread of the handle 4, the cap 22 is fastened to the handle 4, thereby axially fixing the motor 12 to the handle 4. Further, the cap comprised a stop (not shown) in the form of a protrusion extending on the inner surface of the cap 22 for axially blocking a relative movement of the motor 12 relative to the handle 4 in a connected state.

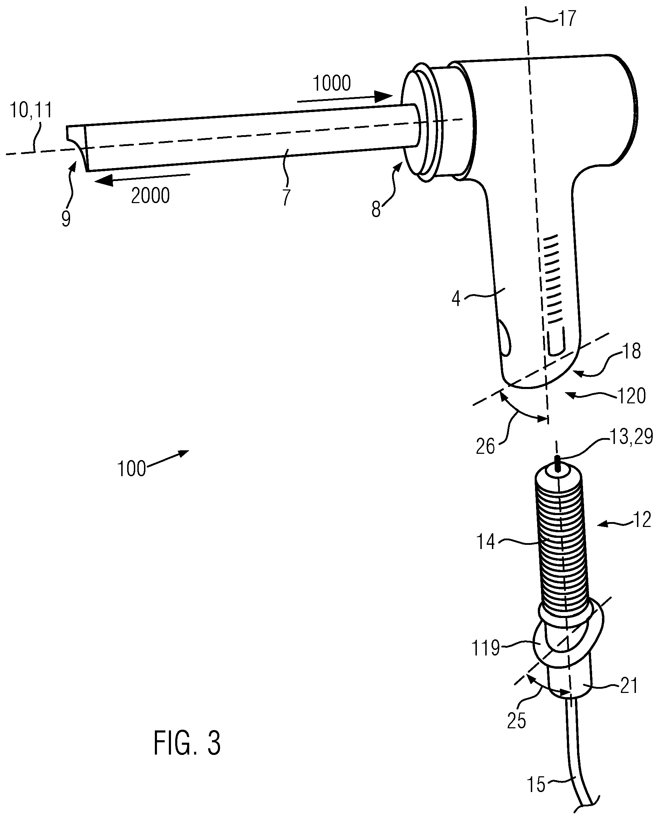

Figure 3 shows an alternative embodiment of a morcellator 100. The morcellator 100 is configured identical to the morcellator 1 as shown in Figure 1 with the exception of the configuration of the motor positioning surface 119 and the handle positioning surface 120. In this embodiment, the motor positioning surface 119 and the handle positioning surface 120 are flat surfaces. The motor positioning surface 119 extends in a plane that is inclined to the longitudinal axis 21 of the motor housing 14 by an angle 25 of 40 degree. Likewise, the handle positioning surface 120 extends in a plane that is inclined to the longitudinal axis 17 of the handle 4 by an angle 26 of 40 degree. Thus, the operator implicitly realizes how to connect the motor 12 and the handle 4 by the inclined motor positioning surface 119 and the handle positioning surface 120. As in Figure 1, the motor 12 is axially fixed to the handle 4 by the cap 22, which is not shown in Figure 3.

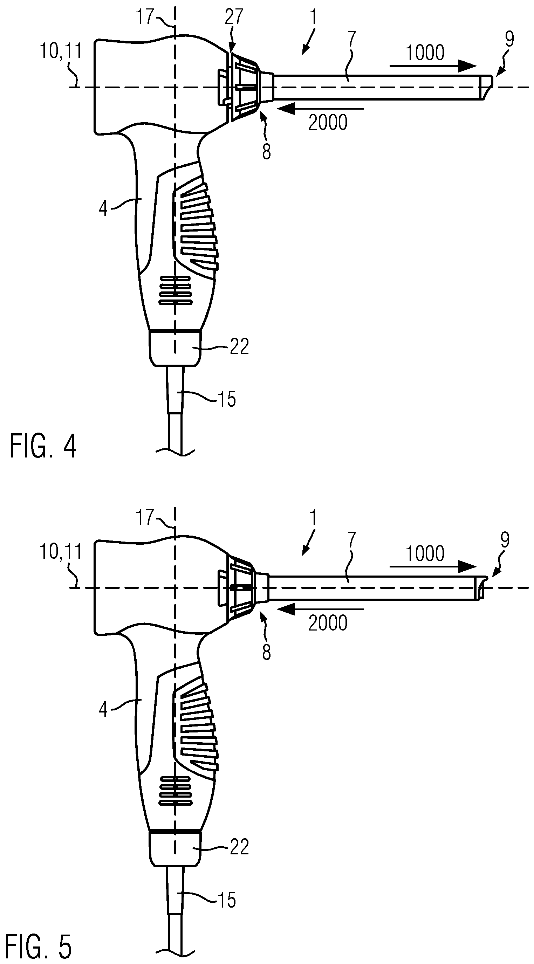

As Figures 4 and 5 show, the outer tube 7 is movable relative to the cutting edge 3 between a cut position and a safe position.

When the outer tube 7 is in the safe position (cf. Figure 4), the distal end 9 of the outer tube 7 is further away from the handle 4 than in the cut position. As Figure 4 shows, there is a small gap 27 of three millimeters, between the outer tube 7 and the handle 4. In this position, cutting of the tissue is not possible.

If the outer tube 7 is in the cut position (cf. Figure 5), the distal end 9 of the outer tube 7 is closer to the handle 4 than in the safe position, such that the gap 27 is closed. In this position cutting of the tissue is possible.

The outer tube 7 is manually switched between the cut position and the safe position. For that purpose, a rotatable cam surface is provided on the handle 4 and on the outer tube 7 a complementary rotatable cam surface is provided, which rotates relative to the rotatable cam surface of the handle 4 by rotating the outer tube 7.

As Figures 6 to 9 show, an insert 29 made of plastic is positioned between the outer tube 7 and the cutting tube 2. The insert 29 forms a guidance and support for the cutting tube 2 within the outer tube 7. Further, the insert 29 comprises a hollow cylindrical bearing part 28, which is torque-proof fixed, in particular pressed, to an inner surface of the outer tube 7. The cylindrical bearing part 28, which functions as a bearing and allows the cutting tube 2 to rotate relative to the outer tube 7.

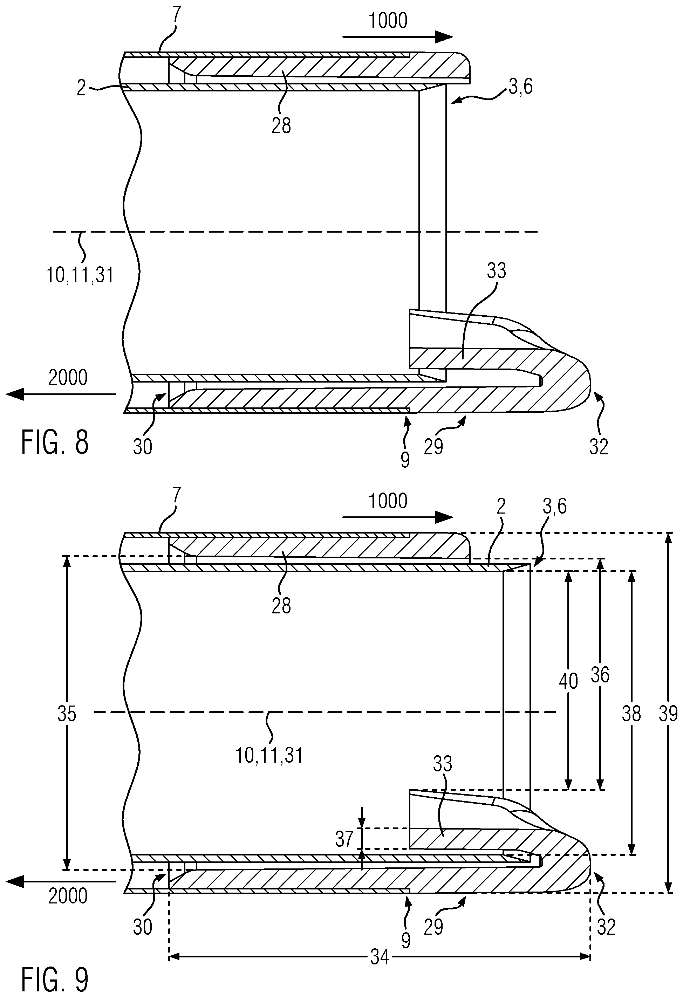

Further, the insert 29, which is positioned close to the distal end 9 of the outer tube 7, extends from a proximal end 30 proximal to the handle 4 along a longitudinal axis 31 to a distal end 32 distal from the handle 4 (cf. Figures 8 and 9). The insert has a length 34 of 20 millimetres. At the proximal end 30, the insert 29 has a proximal inner diameter 35. At the distal end 32, the insert 29 has a distal inner diameter 36. The proximal diameter 35, here seven millimetres, is greater than the distal diameter 36 here five millimetres. Measured in a radial direction, the insert 29 has a radial thickness of two millimetre.

As Figures 6 to 9 show, the insert 29 comprises a lip 33, which is integrally formed with the insert 29 and has a radial thickness 37 of two millimetre, in particular measured from a radial surface of the insert 29 that faces the inside of the cutting tube 2, i. e. that is not in contact with the outer tube 7. The lip 33 exceeds the distal end 32 of the insert 29 along the longitudinal axis 31. Further, the lip 33 exceeds the distal end 9 of the outer tube 7.

The object of the lip 33 is to cover a part of the cutting edge 3, in particular 20 percent of the cutting edge 3, by extending around the cutting edge 3 into an inside of the cutting tube 2. The lip 33 extends along an inner surface of the cutting tube 2 spanning an angle of roughly 70 degree. Thus, the cross-sectional area inside the cutting tube 2 is reduced by the lip 33, resulting in a reduced diameter 40 extending from the inner surface of the cutting tube 2 to the lip 33.

During inserting the cutting tube 2 and the outer tube 7 into the patient, the outer tube 7 is in the safe position. In this position, the insert 29 fully covers the cutting tube 2, in particular the cutting edge 3. Since the distal end 32 of the insert 29 has a rounded edge, the risk of cutting tissue during insertion of the cutting tube 2 and the outer tube 7 into the patient is reduced. After insertion, the outer tube 7 is switched to be in the cut position. The insert 29 is moved together with the outer tube 7 along the longitudinal axis 11 of the outer tube 7 towards the handle 4, thereby closing the gap 27. Thus, the insert 32 now only partially covers the cutting tube 2, in particular the cutting edge 3. The part of the cutting edge 3, which is not covered by the lip 33 is now able cut the tissue if the cutting tube 2 is rotated by the motor 12.

Cutting is only possible with the part of the cutting edge 3, which is not covered by the lip 33. When the cutting edge 3 contacts the tissue, the tissue is cut and transported into the cutting tube 2 out of the patient's body. The lip 33 stabilizes the cut tissue against the rotating cutting tube 2. When the cut tissue contacts the lip 33, the tissue is automatically distanced from the inner surface of the cutting tube 2. Thus, it is avoided that the tissue contacts the inner surface of the rotating cutting tube 2. Thus, the rotating cutting tube 2 cannot cause the tissue to perform a rotary movement but is guided into the cutting tube 2 without being rotated. This reduces the risk that the tissue is torn during transport. Further, segments of tissue are prevented from depositing on the inner wall of the cutting tube 2 and clogging the cutting tube 2, which requires the operation to be stopped. Thus, this embodiment not only reduces the need of exchanging a clogged morcellator 1, 100 by a new morcellator 1, 100 but also reduces the health risk for the patient by preventing the operation from lasting longer because the morcellator 1, 100 has to be changed.

## Claims

1. Morcellator (1, 100) comprising:
a rotatable cutting tube (2) with a cutting edge (3) for cutting and removing human tissue;
a handle (4) for handling the morcellator (1, 100),
wherein the cutting tube (2) is rotatably connected with the handle (4); and
a motor (12) for rotatably driving the cutting tube (2),
**characterized in that**
the motor (12) is reversibly connectable with the handle (4) in a predefined position,
wherein the motor (12) comprises a motor positioning surface (19, 119) and
wherein the handle (4) comprises a handle positioning surface (12, 120),
wherein the motor positioning surface (19, 119) is configured for being in contact with the handle positioning surface (12, 120) in the predefined position for aligning the motor (12) with respect to the handle (4).

2. Morcellator (1, 100) according to claim 1, wherein when the motor (12) is connected to the handle (4), a rotary movement between the motor (12) and the handle (4) is blocked by the contact of the motor positioning surface (19, 119) and the handle positioning surface (12, 120).

3. Morcellator (1, 100) according to claim 1 or 2, wherein the motor positioning surface (19, 119) is in a form-fit with the handle positioning surface (12, 120), when the motor (12) is connected to the handle (4).

4. Morcellator (1, 100) according to one of the preceding claims, wherein the motor (12) comprises a drive shaft (13) configured for driving the cutting tube (2), wherein the motor (12) comprises a motor housing (14) extending at least partially around the drive shaft (13), and wherein the motor housing (14) comprises the motor positioning surface (19, 119), and motor positioning surface (19, 119) extends to an outer surface of morcellator (1, 100).

5. Morcellator (1, 100) according to one of the preceding claims, wherein the handle (4) extends in an axial direction, wherein the handle (4) has a motor receiving cavity (18), wherein the motor (12) is insertable into the motor receiving cavity (18).

6. Morcellator (1, 100) according to claim 5, wherein the handle positioning surface (12, 120) is formed by a surface adjacent to the motor receiving cavity (18).

7. Morcellator (1, 100) according to one of the preceding claims, wherein the handle positioning surface (12, 120) extends in a plane that is at least locally inclined to a longitudinal axis (17) of the handle (4), wherein the motor positioning surface (19, 119) extends in a plane that is at least locally inclined to a longitudinal axis (21) of the motor housing (14), and wherein the handle positioning surface (12, 120) and the motor positioning surface (19, 119) are at inclined by the same degree in corresponding locations.

8. Morcellator (1, 100) according to one of the preceding claims, wherein the motor positioning surface (19, 119) has a repeating structure, in particular extending in the circumferential direction of the motor housing (14), and wherein the handle positioning surface (12, 120) has a repeating structure, in particular extending in the circumferential direction of the handle (4).

9. Morcellator (1, 100) according to one of the preceding claims, further comprising a cap (22) for, in particular axially, fixing the motor (12) with respect to the handle (4) in a detachable manner.

10. Morcellator (1, 100) according to the preamble of claim 1 or any of the proceedings claims, further comprising:
a stationary outer tube (7) fixed to the handle (4), wherein the outer tube (7) is configured to extend around the cutting tube (2); and
an insert (29), wherein the insert (29) is positioned between the outer tube (7) and the cutting tube (2).

11. Morcellator (1, 100) according to claim 10, wherein the insert (29) provides a bearing in between the outer tube (7) and cutting tube (2).

12. Morcellator (1, 100) according to claim 10 or 11, wherein the cutting tube (2) has a proximal end (5) proximal to the handle (4), and a distal end (6) with the cutting edge (3) distal from the handle (4),
wherein the proximal end (5) of the cutting tube (2) has the same diameter as the distal end (6) of the cutting tube (2).

13. Morcellator (1, 100) according to one of the claims 10 to 12, wherein the insert (29) comprises a lip (33), which covers, at least part of, the cutting edge (3) and extends around the cutting edge (3) into an inside of the cutting tube (2).

14. Morcellator (1, 100) according to claim 13, wherein the lip (33) protrudes at least 2 millimetres over a distal end of the outer tube (7).

15. Morcellator (1, 100) according to one of the claims 10 to 14, wherein outer tube (7) and cutting tube (2) are axially movable relative to each other between a safe position and a cut position, wherein the insert (32) fully covers the cutting tube (2), in the safe position.
